# EUROPEAN PATENT SPECIFICATION

(11) **EP 2 501 296 B1**
(45) Date of publication and mention of the grant of the patent: **09.01.2019**
(21) Application number: 10832222.3
(22) Date of filing: 19.11.2010
(51) Int. Cl.: A61B 8/00, A61B 8/08

(54) **SPINOUS NAVIGATION SYSTEM AND ASSOCIATED METHODS**
DORNFORTSATZ-NAVIGATIONSSYSTEM UND ZUGEHÖRIGE VERFAHREN
SYSTÈME DE NAVIGATION VERTÉBRAUX ET PROCÉDÉS ASSOCIÉS

(30) Priority: 19.11.2009 US 621934
(43) Date of publication of application: 26.09.2012
(73) Proprietor: Globus Medical, Inc., Audubon, PA 19403 (US)
(72) Inventor: SUH, Sean, Plymouth Meeting, PA 19462 (US); IOTT, Andrew, Villanova, PA 19085 (US); LEONARD, Robert, Furlong, PA 18925 (US)
(74) Representative: Morabito, Sara
(86) International application number: PCT/US2010/057330
(87) International publication number: WO 2011/063176

(56) References cited:
- WO-A2-2009/105616
- CN-A- 1 745 721
- US-A- 5 088 208
- US-A1- 2005 080 320
- US-A1- 2008 004 615
- US-A1- 2008 091 109
- US-A1- 2008 119 737
- US-A1- 2008 243 142
- US-B1- 6 474 341
- KURS ET AL.: 'Wireless Power Transfer via Strongly Coupled Magnetic Resonances [Retrieved from ScienceXpress', [Online] 07 June 2007, XP008157420 Retrieved from the Internet: <URL:http:/www.signallake.com/innovation/ WirelessPowerTransmission060707.pdf>

## Description

### FIELD OF THE INVENTION

The present disclosure generally relates to spinal navigation and more particularly, in one or more embodiments, to using a sonar probe for navigation through internal body structures to access the spine. The invention is defined by the appended claims. All other embodiments are exemplary.

### BACKGROUND

Conventional methods for accessing spinal discs of the lumbar plexus during surgery include dilation of the psoas muscle. Dilation is typically accomplished with cannula. Drawbacks to such conventional methods include damage to nerves as the cannula traverses the psoas muscle.

Neuro-monitoring has been developed to overcome the drawbacks of such conventional methods. Neuro-monitoring methods include use of a stimulation clip attached to the cannula. The stimulation clip may be stimulated to produce signals as the cannula advances through the psoas muscle. The return signals may be monitored for the presence of a nerve. Drawbacks to neuro-monitoring include that such methods do not allow for visual identification of the nerves. Additional drawbacks to neuro-monitoring include inefficiencies in determining the distance from the cannula to the nerve. US2008/0119737 discloses an integrated ultrasound and nerve stimulation system.

Thus, there is a need for improved systems and methods of accessing spinal discs. Further needs include improved systems and methods for identifying nerves when accessing spinal discs.

### SUMMARY

An embodiment of the present invention provides a system for accessing a spinal disc disposed within internal body structures. The system includes a sonar probe. The sonar probe emits sound waves into the internal body structures to create return signals. The system also includes a retractor and a monitoring system. The monitoring system includes an ultrasound imaging screen. The ultrasound imaging screen displays images of the internal body structures from the return signals. The ultrasound imaging screen also provides a visual identification of a nerve when disposed in the internal body structures.

The features and advantages of the present invention will be readily apparent to those skilled in the art. While numerous changes may be made by those skilled in the art, such changes are within the spirit of the invention.

### BRIEF DESCRIPTION OF THE DRAWINGS

FIG. 1 is an embodiment of a spinal navigation system with a sonar probe in psoas muscle as shown in an ultrasound imaging screen;
FIG. 2 is a cross sectional view of a spinal disc;
FIG. 3 is an embodiment of a spinal navigation system with a sonar probe emitting lateral sound waves in muscle;
FIG. 4 is an embodiment as shown on an ultrasound imaging screen indicating the lateral distance to the sonar probe from the nerve;
FIG. 5 is an embodiment of a spinal navigation system with a sonar probe emitting axial sound waves in muscle;
FIG. 6 is an embodiment as shown on an ultrasound imaging screen indicating the axial distance to the sonar probe from the nerve;
FIG. 7 is an embodiment of a spinal navigation system with a bi-directional sonar probe in muscle;
FIG. 8 is an embodiment of a spinal navigation system with a bi-directional sonar probe with a retractor;
FIG. 9 is an embodiment of a spinal navigation system with a bi-directional sonar probe with multiple retractors for sequential dilation;
FIG. 10 is an embodiment of a sonar probe with a wire and a retractor;
FIG. 11 is an embodiment of a wireless sonar probe disposed within a retractor;
FIG. 12 is an embodiment of a wireless sonar probe;
FIG. 13 is an embodiment of a spinal navigation system with a sonar probe and a nucleus removal instrument;
FIG. 14 is an embodiment of an ultrasound imaging screen showing a removed portion of the nucleus;
FIG. 15 is an embodiment of a spinal navigation system with a sonar probe having an endoscopic camera;
FIG. 16 is an embodiment of a spinal navigation system with a sonar probe in muscle and with the sonar probe having an endoscopic camera;
FIG. 17 is an embodiment of a camera screen showing an image of a muscle and annulus fibrosus;
FIG. 18 is an embodiment of a retractor having an opening.

### DETAILED DESCRIPTION OF THE ILLUSTRATED EMBODIMENTS

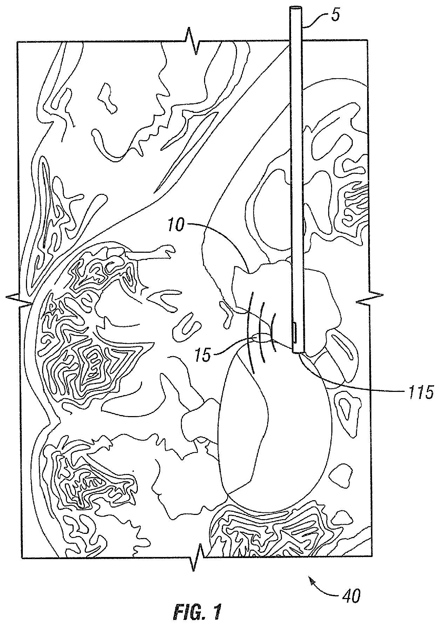

FIG. 1 shows an embodiment of a spinal navigation system comprising sonar probe 5 navigating through muscle 10. Sonar probe 5 allows visual identification of nerve 15 and a determination of the distance from tip 115 of sonar probe 5 to nerve 15, which allows an operator of sonar probe 5 to navigate sonar probe 5 through muscle 10 without damaging nerve 15. Without limitation, by allowing visual identification of nerve 15 and a determination of the distance from tip 115 to nerve 15, sonar probe 5 allows for estimation of the distance to nerve 15 prior to insertion of retractors (not illustrated). It is to be understood that FIG. 1 shows sonar probe 5 superimposed on ultrasound imaging screen 40 for illustrative purposes only. In an embodiment, muscle 10 comprises psoas muscle.

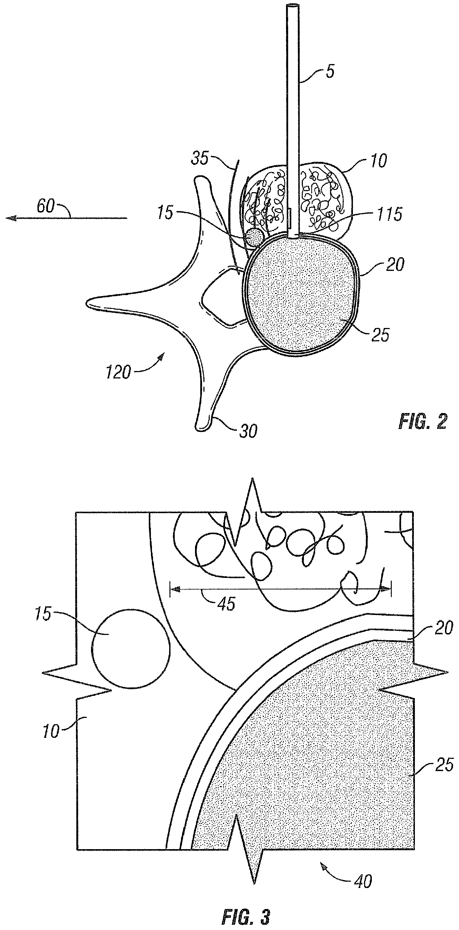

FIG. 2 illustrates an embodiment of a spinal navigation system comprising sonar probe 5 navigating through muscle 10 to spinal disc 120. Spinal disc 120 includes annulus fibrosus 20, nucleus pulposus 25, and spinous process 30. As shown in FIG. 2, sonar probe 5 emits sound waves 35 from tip 115 into internal body structures. Sound waves 35 are emitted at frequencies sufficient to image internal structures of the body such as muscle 10 and spinal disc 120. In the embodiment as shown in FIG. 2, sonar probe 5 emits sound waves 35 in a lateral direction 60 in relation to sonar probe 5. Sound waves 35 are emitted and contact nerve 15 as sonar probe 5 traverses internal body structures including muscle 10. It is to be understood that sonar probe 5 emits sounds waves 35 continuously as sonar probe 5 traverses muscle 10 to spinal disc 120. In alternative embodiments, sonar probe 5 emits sounds waves 35 intermittently. The sound waves 35 return, and the signals from the returning sound waves (not illustrated) are collected by sonar probe 5 and transmitted to a signal receiver (not illustrated). A monitoring system (not illustrated) uses the signals received by the signal receiver to create an image of internal body structures including muscle 10, nerve 15, and spinal disc 120. The signal receiver includes any device suitable for receiving a signal from a sonar probe. The signal receiver may be located at any suitable location. In an embodiment, the signal receiver is located in proximity to the patient upon which sonar probe 5 is being used. For instance, in an embodiment, the signal receiver is located in the operating room with the patient. The monitoring system comprises any devices and methods suitable for providing an image from signals created by sound waves 35 contacting internal body structures. In an embodiment, the monitoring system comprises an ultrasound device. The ultrasound device includes any device suitable for ultrasonography. It is to be understood that ultrasonography refers to diagnostic imaging in which ultrasound is used to image internal body structures. The monitoring system may be located at any suitable location. In an embodiment, the monitoring system is located in proximity to the patient upon which sonar probe 5 is being used. For instance, in an embodiment, the monitoring system is located in the operating room with the patient. The monitoring system may include an ultrasound imaging screen 40, as shown in FIG. 3. Ultrasound imaging screen 40 includes any screen suitable for displaying the image of internal body structures such as spinal disc 120. In an embodiment, the monitoring system comprises the signal receiver.

FIG. 3 illustrates an embodiment of the spinal navigation system in which ultrasound imaging screen 40 displays an image of muscle 10, nerve 15, annulus fibrosus 20, and nucleus pulposus 25 as the sonar probe 5 shown in FIG. 3 navigates through muscle 10 and emits sound waves 35 in a lateral direction 60. As shown in FIG. 3, the monitoring system allows for the lateral distance 45 from sonar probe 5 to nerve 15 to be determined and visualized on ultrasound imaging screen 40. Lateral distance 45 may be determined by any suitable distance determination techniques used with monitoring systems such as ultrasound devices. In an embodiment, continually monitoring the determined lateral distance 45 of sonar probe 5 from nerve 15 allows the spinal navigation system to safely navigate the muscle 10 without damaging nerve 15 and access spinal disc 120. For instance, during movement of sonar probe 5 through muscle 10 to spinal disc 120, the operator of sonar probe 5 may monitor the images on ultrasound imaging screen 40 and also monitor the determined lateral distances 45 to any nerves 15. Such monitoring allows the operator to control movement of sonar probe 5 through muscle 10 and other internal body structures without damaging nerves 15.

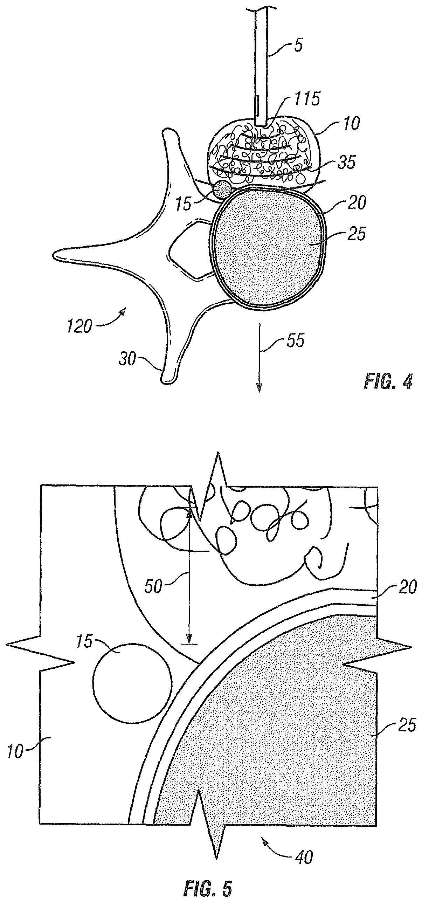

FIG. 4 illustrates an embodiment of a spinal navigation system comprising sonar probe 5 navigating through muscle 10 to spinal disc 120 with sonar probe 5 emitting sound waves 35 from tip 115 in an axial direction 55 in relation to sonar probe 5. Sound waves 35 are emitted and contact nerve 15 as sonar probe 5 traverses muscle 10. From the return signal, the monitoring system creates an image of internal body structures such as spinal disc 120 and nerve 15. FIG. 5 illustrates an embodiment of an image created by the monitoring system from the sound waves 35 emitted in the axial direction 55. As shown in FIG. 5, the monitoring system allows for the axial distance 50 from sonar probe 5 to nerve 15 to be determined and visualized on ultrasound imaging screen 40. Axial distance 50 may be determined by any suitable distance determination techniques used with monitoring systems such as ultrasound devices. In an embodiment, continually monitoring the determined axial distance 50 of sonar probe 5 from nerve 15 allows the spinal navigation system to safely navigate the sensor probe 5 through the muscle 10 without damaging nerve 15 and thereby access spinal disc 120. For instance, during movement of sonar probe 5 through muscle 10 to spinal disc 120, the operator of sonar probe 5 may monitor the images on ultrasound imaging screen 40 and also the determined axial distances 50 to any nerves 15. Such monitoring allows the operator to control movement of sonar probe 5 through muscle 10 and other internal body structures without damaging nerves 15.

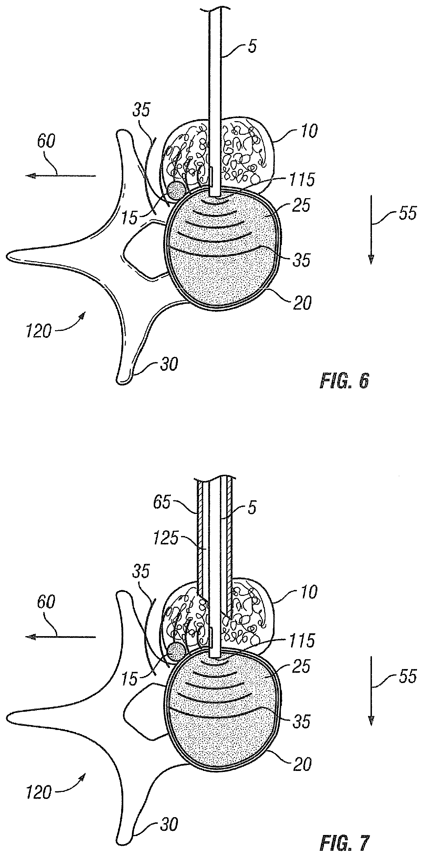

FIG. 6 illustrates an embodiment of a spinal navigation system in which sonar probe 5 is a bi-directional probe and emits sound waves 35 from tip 115 in axial direction 55 and lateral direction 60. It is to be understood that FIG. 6 shows sound waves 35 emitted in only one lateral direction 60 for illustrative purposes only and that sonar probe 5 may also emit sound waves 35 in the opposing lateral direction. The bi-directional sonar probe 5 allows for determination of both lateral distance 45 and axial distance 50 to nerve 15 and visualization on ultrasound imaging screen 40 (not illustrated). In an embodiment, continually monitoring the images of the internal body structures and the determined lateral distance 45 and axial distance 50 on ultrasound imaging screen 40 allows the spinal navigation system to safely navigate muscle 10 and other internal body structures without damaging nerve 15 and thereby access spinal disc 120. For instance, during movement of sonar probe 5 through muscle 10 to spinal disc 120, the operator of sonar probe 5 may monitor the images on ultrasound imaging screen and also the determined lateral distances 45 and axial distances 50 to any nerves 15, which allows the operator to control movement of sonar probe 5 through muscle 10 and other internal body structures without damaging nerves 15.

FIG. 7 illustrates an embodiment of a spinal navigation system comprising sonar probe 5 and retractor 65. Retractor 65 may, for instance, be mounted to sonar probe 5. Sonar probe 5 is disposed within interior 125 of retractor 65. In such an embodiment, sonar probe 5 may be a bi-directional probe emitting sound waves 35 from tip 115 in axial direction 55 and lateral direction 60.

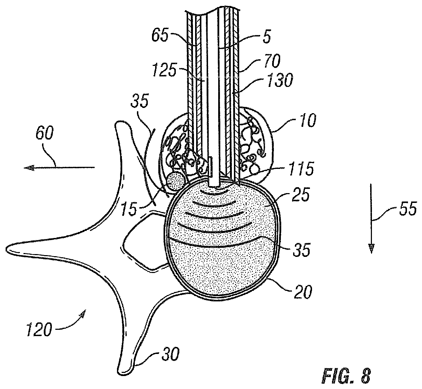

FIG. 8 illustrates an embodiment of a spinal navigation system comprising sonar probe 5 and retractors 65, 70. Retractor 65 may, for instance, be mounted to sonar probe 5, and retractor 70 may be slid over retractor 65. In such an embodiment, sonar probe 5 is a bi-directional probe emitting sound waves 35 from tip 115 in axial direction 55 and lateral direction 60.

In the embodiments illustrated in FIG. 7 and FIG. 8, sonar probe 5 allows for constant monitoring of nerve 15 during dilation by retractor 65 and sequential dilation by retractors 65, 70. In some embodiments, sonar probe 5 is the initial dilator. It is to be understood that the spinal navigation system is not limited to retractors 65, 70 but may include any number of retractors suitable to achieve a desired dilation. In an embodiment, after navigating sonar probe 5 through muscle 10, retractor 65 is guided through muscle 10 to spinal disc 120 with sonar probe 5 disposed within interior 125. In embodiments, during movement of retractor 65 through muscle 10 to spinal disc 120, the operator of retractor 65 may monitor the images on ultrasound imaging screen 40 and also the determined lateral distances 45 and axial distances 50 from retractor 65 to any nerves 15, which allows the operator to direct movement of retractor 65 through muscle 10 without damaging any nerves 15. After dilation with retractor 65, retractor 70 is guided through muscle 10 to spinal disc 120 with sonar probe 5 and retractor 65 disposed within interior 130 of retractor 70. In some embodiments, during movement of retractor 70 through muscle 10 to spinal disc 120, the operator of retractor 70 may monitor the images on ultrasound imaging screen 40 and also the determined lateral distances 45 and axial distances 50 from retractor 70 to any nerves 15, which allows the operator to direct movement of retractor 70 through muscle 10 without damaging any nerves 15.

In alternative embodiments (not illustrated), retractor 65 or retractor 70 may have a sonar probe 5 mounted thereto. In such alternative embodiments, sonar probe 5 may slide over the retractor 65 or retractor 70 for dilation and navigation. In other alternative embodiments (not illustrated), sonar probes 5 are mounted to more than one retractor. In such other alternative embodiments, the sonar probes 5 slide over the retractors for sequential dilation.

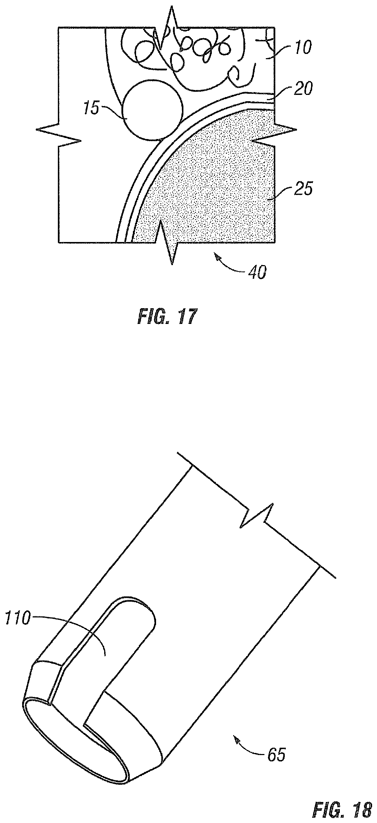

Retractors 65, 70 may comprise any device that may be used to hold back edges of an incision. In an embodiment, retractors 65, 70 comprise a cannula. FIG. 18 illustrates an embodiment in which retractor 65 comprises a cannula having an opening 110. In some embodiments, opening 110 is positioned to allow sound waves 35 to pass therethrough in a desired direction. In alternative embodiments (not illustrated), retractor 65 has more than one opening 110. In another embodiment (not illustrated), retractor 65 and/or retractor 70 may comprise multiple blades. In some embodiments, the multiple blades may be expanded.

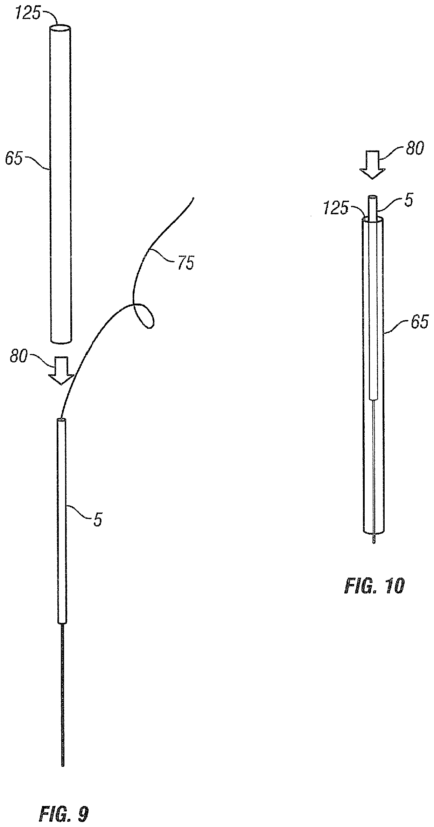

FIG. 9 illustrates an embodiment of sonar probe 5 in which sonar probe 5 comprises wire 75. FIG. 9 illustrates an embodiment of the spinal navigation system in which retractor 65 is slidable over sonar probe 5. Arrow 80 is for illustrative purposes only to show the direction of movement of retractor 65 over sonar probe 5.

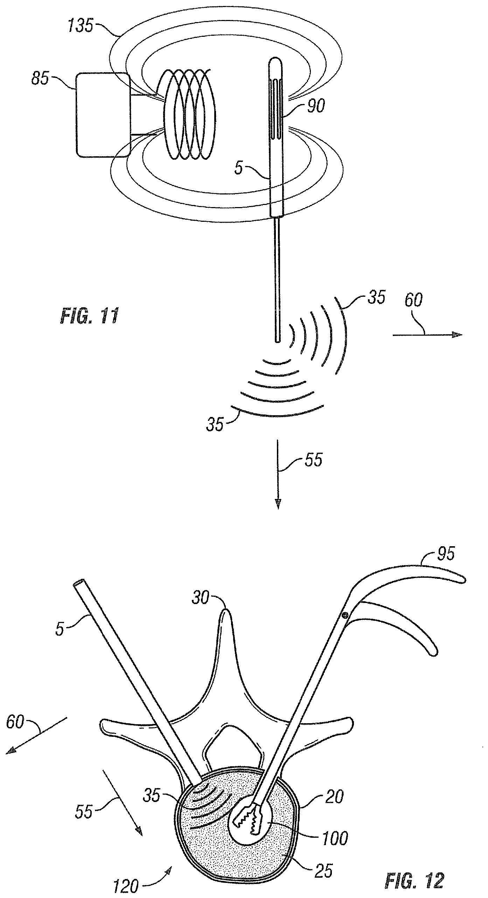

FIG. 10 illustrates an embodiment of sonar probe 5 in which sonar probe 5 is a wireless probe. FIG. 10 also illustrates an embodiment of the spinal navigation system in which retractor 65 is slidable over sonar probe 5. A wireless sonar probe 5 may be powered by any suitable power source such as battery power, magnetic induction resonance, and the like. FIG. 12 illustrates an embodiment of wireless sonar probe 5 powered by magnetic induction resonance. Any magnetic induction resonance method suitable for use with a surgical probe may be used. In an embodiment as illustrated in FIG. 11, sonar probe 5 is powered through magnetic induction resonance 135 between source 85 and receiver 90. It is to be understood that the size shown of source 85 in relation to sonar probe 5 is for illustrative purposes only. In an embodiment (not illustrated), source 85 is disposed in or on sonar probe 5. As shown in FIG. 11, receiver 90 is contained within or alternatively on sonar probe 5. As illustrated, the embodiment of FIG. 11 is a wireless sonar probe 5 powered by magnetic induction resonance and that is a bi-directional probe.

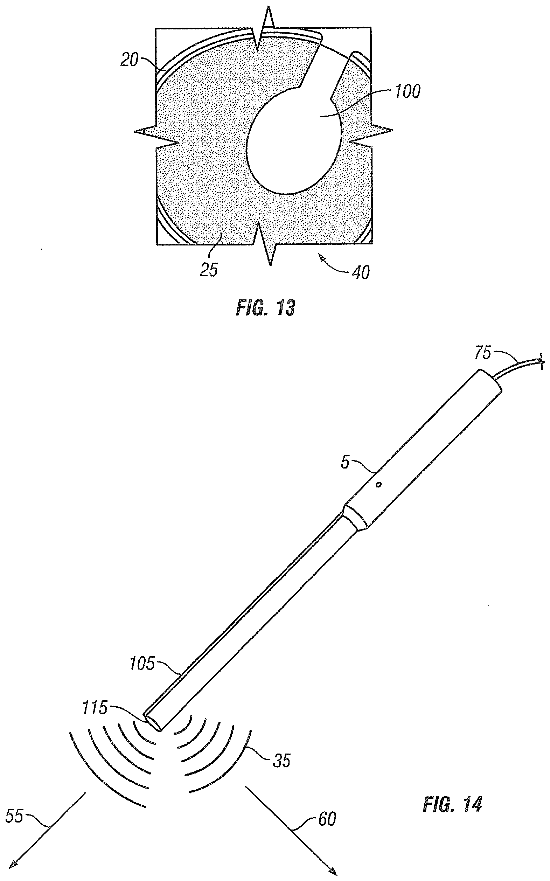

FIG. 12 illustrates an embodiment of the spinal navigation system comprising sonar probe 5 and nucleus removal instrument 95. Nucleus removal instrument 95 comprises any device suitable for removing nucleus pulposus 25. Without limitation, examples of suitable nucleus removal instruments 95 include a curette, a cobb elevator, a box cutter, and the like. In an embodiment, the operator monitors the movement of sonar probe 5 through muscle 10 (not illustrated) to spinal disc 120 using the images on ultrasound imaging screen 40 (not illustrated) generated by the monitoring system from the return signals of sound waves 35. After sonar probe 5 is in a desired position, the operator monitors the movement of one or more retractors (not illustrated) to spinal disc 120 using the images on ultrasound imaging screen 40 (not illustrated). The retractor or retractors are used to provide a sufficient dilation to allow nucleus removal instrument 95 to be moved through the dilation to nucleus pulposus 25. In alternative embodiments (not illustrated), sonar probe 5 is used with one or more retractors mounted to sonar probe 5 (not illustrated). Nucleus removal instrument 95 is used to remove any desired portion of nucleus pulposus 25. During removal of nucleus pulposus 25, sound waves 35 are continued to be emitted with the return signals allowing the monitoring system to image the nucleus pulposus 25. FIG. 13 illustrates an image on ultrasound imaging screen 40 of nucleus pulposus 25 with removed portion of nucleus 100. In an embodiment as illustrated in FIGS. 12 and 13, such imaging of nucleus pulposus 25 allows an operator to detect the location of removed portion of nucleus 100. In embodiments, such imaging also allows an operator to monitor the amount of removed portion of nucleus 100. Without limitation, such imaging allows an operator to visually monitor and direct the amount and location of the portions of nucleus pulposus 25 to be removed.

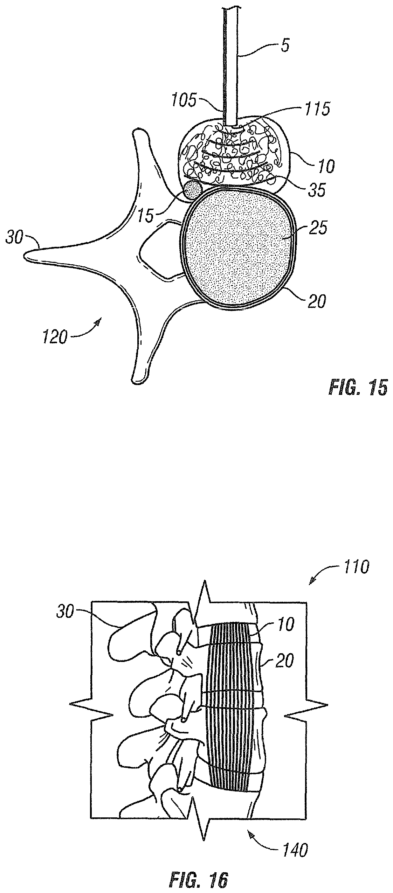

FIGS. 14 and 15 illustrate an embodiment of sonar probe 5 in which sonar probe 5 comprises endoscopic camera 105. Endoscopic camera 105 may include any camera suitable for use with endoscopy. In alternative embodiments (not illustrated), sonar probe 5 comprises more than one endoscopic camera 105. In an embodiment, sonar probe 5 has endoscopic camera 105 disposed at tip 115. In alternative embodiments (not illustrated), endoscopic camera 105 is disposed near tip 115. FIG. 16 illustrates a camera screen 140 showing a view through endoscopic camera 105 of the embodiment of FIG. 15. In an embodiment, endoscopic camera 105 provides an operator of sonar probe 5 visual monitoring when accessing muscle 10. FIG. 17 illustrates an ultrasound imaging screen 40 providing an image of return signals from sonar probe 5 of the embodiment of FIG. 15. Without limitation, sonar probe 5 comprising endoscopic camera 105 allows real time visual monitoring as sonar probe 5 proceeds from accessing muscle 10 as facilitated by endoscopic camera 105 to dilation of muscle 10 as facilitated by sound waves 35 of sonar probe 5.

In an embodiment, nerves 15 are plexus nerves located in the lumbar plexus. It is to be understood that the spinal navigation system is not limited to accessing spinal discs 120 in the lumbar plexus to avoid damaging nerves 15 comprising plexus nerves. In alternative embodiments, the spinal navigation system may be used to access spinal discs 120 in any portion of the spine. In such alternative embodiments, muscle 10 is not limited to psoas muscle but also includes any muscle or tissue that must be traversed to access the desired spinal disc 120.

While it is apparent that the invention disclosed herein is well calculated to fulfill the objects stated above, it will be appreciated that numerous modifications and embodiments may be devised by those skilled in the art.

In a further embodiment it is provided a method for accessing a spinal disc disposed within internal body structures, comprising: navigating a sonar probe through the internal body structures, wherein the sonar probe emits sound waves into the internal body structures to create return signals; and monitoring the navigation of the sonar probe through the internal body structures, wherein monitoring comprises providing images of the internal body structures from the return signal on an ultrasound imaging screen; navigating a retractor through the internal body structures; and identifying a nerve when disposed in the internal body structures.

The method further can comprise determining a distance from the nerve to the sonar probe. The method can further provide for mounting the retractor to the sonar probe.

The method can further provide for monitoring the navigation of the retractor through the internal body structures, wherein monitoring comprises providing images of the internal body structures from the return signal on an ultrasound imaging screen.

In a version the sonar probe is powered by magnetic induction resonance.

In a version the retractor comprises multiple blades.

## Claims

1. A system for accessing a spinal disc disposed within internal body structures (10, 15, 120), comprising:
- a sonar probe (5), wherein the sonar probe (5) is configured for emitting sound waves (35) into the internal body structures (10, 15, 120) to create return signals;
- a monitoring system, wherein the monitoring system comprises an ultrasonography imaging screen, and wherein the ultrasonography imaging screen is arranged to display images of the internal body structures from the return signals, and further wherein the ultrasonography imaging screen is configured to provide a visual identification of a nerve when disposed in the internal body structures (10, 15, 120), **characterized in that** the system comprises a retractor (65,70) and
- wherein the sonar probe (5) includes an endoscopic camera (105) and wherein the endoscopic camera (105) provides real-time visual monitoring as the sonar probe (5) proceeds from accessing muscle to dilation of muscle as facilitated by sound waves of the sonar probe.

2. The system of claim 1, wherein the sonar probe (5) comprises a tip (115), and wherein the sound waves are emitted from the tip (115).

3. The system of claim 1, or 2, wherein the sonar probe (5) emits the sound waves in an axial direction (55) and a lateral direction (60) in relation to the sonar probe (5).

4. The system of any one of the preceding claims, wherein the monitoring system is arranged to determine a distance(45) from the nerve (15) to the sonar probe (5).

5. The system of any one of the preceding claims, wherein the ultrasound imaging screen is arranged to display the images of the internal body structures (10, 15, 120) as the sonar probe (5) traverses the internal body structures.

6. The system of any one of the preceding claims, wherein the retractor (65, 70) comprises a cannula.

7. The system of the preceding claim, wherein the cannula comprises at least one opening (110).

8. The system of any one of the preceding claims, wherein the retractor (65, 70)is mounted to the sonar probe (5).

9. The system of any one of the preceding claims, wherein the ultrasound imaging screen is arranged to display the images of the internal body structures as the retractor traverses the internal body structures.

10. The system of any one of the preceding claims, wherein the monitoring system comprises an ultrasound device.

11. The system of any one of the preceding claims, wherein the sonar probe comprises a wireless probe, and wherein the sonar probe is powered by magnetic induction resonance.

12. The system of claim any one of the preceding claims, further comprising a nucleus removal instrument (95).

13. The system of claim any one of the preceding claims, wherein the retractor (65, 70) comprises multiple blades.

14. The system of the preceding claim, wherein the multiple blades are expandable.

## Patentansprüche

1. System zum Zugang auf eine Bandscheibe, die innerhalb von inneren Körperstrukturen (10, 15, 120) angeordnet ist, umfassend:
- eine Sonarsonde (5), wobei die Sonarsonde (5) zum Emittieren von Schallwellen (35) in die inneren Körperstrukturen (10, 15, 120) eingerichtet ist, um Echosignale zu erzeugen;
- ein Überwachungssystem, wobei das Überwachungssystem einen Ultrasonographie-Bildschirm aufweist, und wobei der Ultrasonographie-Bildschirm angeordnet ist, um Bilder der inneren Körperstrukturen aus den Echosignalen darzustellen, und wobei ferner der Ultrasonographie-Bildschirm eingerichtet ist, um eine visuelle Identifizierung eines Nervs vorzusehen, wenn er in den inneren Körperstrukturen (10, 15, 120) angeordnet ist, **dadurch gekennzeichnet, dass** das System einen Retraktor (65,70) aufweist, und
- wobei die Sonarsonde (5) eine endoskopische Kamera (105) umfasst, und wobei die endoskopische Kamera (105) eine visuelle Echtzeitüberwachung vorsieht, wenn die Sonarsonde (5) den Zugang auf einen Muskel durch Muskeldehnung mit Unterstützung von Schallwellen der Sonarsonde fortsetzt.

2. System nach Anspruch 1, wobei die Sonarsonde (5) eine Spitze (115) aufweist, und wobei die Schallwellen von der Spitze (115) emittiert werden.

3. System nach Anspruch 1 oder 2, wobei die Sonarsonde (5) die Schallwellen in eine axiale Richtung (55) und eine seitliche Richtung (60) in Bezug auf die Sonarsonde (5) emittiert.

4. System nach einem der vorhergehenden Ansprüche, wobei das Überwachungssystem angeordnet ist, um einen Abstand (45) vom Nerv (15) zur Sonarsonde (5) zu bestimmen.

5. System nach einem der vorhergehenden Ansprüche, wobei der Ultrasonographie-Bildschirm angeordnet ist, um die Bilder der inneren Körperstrukturen (10, 15, 120) darzustellen, wenn die Sonarsonde (5) die inneren Körperstrukturen durchläuft.

6. System nach einem der vorhergehenden Ansprüche, wobei der Retraktor (65, 70) eine Kanüle aufweist.

7. System nach dem vorhergehenden Anspruch, wobei die Kanüle mindestens eine Öffnung (110) aufweist.

8. System nach einem der vorhergehenden Ansprüche, wobei der Retraktor (65, 70) an der Sonarsonde (5) befestigt ist.

9. System nach einem der vorhergehenden Ansprüche, wobei der Ultrasonographie-Bildschirm angeordnet ist, um die Bilder der inneren Körperstrukturen darzustellen, wenn der Retraktor die inneren Körperstrukturen durchläuft.

10. System nach einem der vorhergehenden Ansprüche, wobei das Überwachungssystem eine Ultraschallvorrichtung aufweist.

11. System nach einem der vorhergehenden Ansprüche, wobei die Sonarsonde eine drahtlose Sonde aufweist, und wobei die Sonarsonde durch magnetische Induktionsresonanz betrieben wird.

12. System nach einem der vorhergehenden Ansprüche, das ferner ein Nukleus-Entnahmeinstrument (95) aufweist.

13. System nach einem der vorhergehenden Ansprüche, wobei der Retraktor (65, 70) mehrere Klingen aufweist.

14. System nach dem vorhergehenden Anspruch, wobei die mehreren Klingen erweiterbar sind.

## Revendications

1. Système pour accéder à un disque intervertébral disposé dans des structures corporelles internes (10, 15, 120), comprenant :
- une sonde à sonar (5), dans lequel la sonde à sonar (5) est configurée pour émettre des ondes sonores (35) dans les structures corporelles internes (10, 15, 120) pour créer des signaux de retour ;
- un système de contrôle, dans lequel le système de contrôle comprend un écran d'imagerie à ultrasons, et dans lequel l'écran d'imagerie à ultrasons est agencé pour afficher des images des structures corporelles internes à partir des signaux de retour, et en outre dans lequel l'écran d'imagerie à ultrasons est configuré pour fournir une identification visuelle d'un nerf quand il est disposé dans les structures corporelles internes (10, 15, 120), **caractérisé en ce que** le système comprend un élément de rétraction (65, 70) et
- dans lequel la sonde à sonar (5) inclut une caméra endoscopique (105) et dans lequel la caméra endoscopique (105) fournit un contrôle en temps réel quand la sonde à sonar (5) avance de l'accès au muscle à la dilatation du muscle tel que facilité par des ondes sonores de la sonde à sonar.

2. Système selon la revendication 1, dans lequel la sonde à sonar (5) comprend une pointe (115), et dans lequel les ondes sonores sont émises à partir de la pointe (115).

3. Système selon la revendication 1, ou 2, dans lequel la sonde à sonar (5) émet les ondes sonores dans une direction axiale (55) et une direction latérale (60) par rapport à la sonde à sonar (5).

4. Système selon l'une quelconque des revendications précédentes, dans lequel le système de contrôle est agencé pour déterminer une distance (45) du nerf(15) à la sonde à sonar (5).

5. Système selon l'une quelconque des revendications précédentes, dans lequel l'écran d'imagerie à ultrasons est agencé pour afficher les images des structures corporelles internes (10, 15, 120) quand la sonde à sonar (5) traverse les structures corporelles internes.

6. Système selon l'une quelconque des revendications précédentes, dans lequel l'élément de rétraction (65, 70) comprend une canule.

7. Système selon la revendication précédente, dans lequel la canule comprend au moins une ouverture (110).

8. Système selon l'une quelconque des revendications précédentes, dans lequel l'élément de rétraction (65, 70) est monté sur la sonde à sonar (5).

9. Système selon l'une quelconque des revendications précédentes, dans lequel l'écran d'imagerie à ultrasons est agencé pour afficher les images des structures corporelles internes quand l'élément de rétraction traverse les structures corporelles internes.

10. Système selon l'une quelconque des revendications précédentes, dans lequel le système de contrôle comprend un dispositif à ultrasons.

11. Système selon l'une quelconque des revendications précédentes, dans lequel la sonde à sonar comprend une sonde sans fil, et dans lequel la sonde à sonar est alimentée par résonance à induction magnétique.

12. Système selon l'une quelconque des revendications précédentes, comprenant en outre un instrument de retrait de noyau (95).

13. Système selon l'une quelconque des revendications précédentes, dans lequel l'élément de rétraction (65, 70) comprend de multiples lames.

14. Système selon la revendication précédente, dans lequel les multiples lames sont extensibles.
